# EUROPÄISCHE PATENTSCHRIFT

(11) **EP 2 849 642 B1**
(45) Veröffentlichungstag und Bekanntmachung des Hinweises auf die Patenterteilung: **29.04.2020**
(21) Anmeldenummer: 13737792.5
(22) Anmeldetag: 08.05.2013
(51) Int. Cl.: A61B 5/055, A61B 5/00

(54) **KOPFHALTERUNG ZUR FIXIERUNG DES KOPFES VON PATIENTEN**
HEAD RESTRAINER FOR IMMOBILISING THE HEAD OF PATIENTS
DISPOSITIF DE FIXATION DE TÊTE PERMETTANT D'IMMOBILISER LA TÊTE D'UN PATIENT

(30) Priorität: 15.05.2012 DE 102012009584
(43) Veröffentlichungstag der Anmeldung: 25.03.2015
(73) Patentinhaber: Noras, Hubert, 97082 Würzburg (DE)
(72) Erfinder: Noras, Hubert, 97082 Würzburg (DE)
(74) Vertreter: Pöhner, Wilfried Anton
(86) Internationale Anmeldenummer: PCT/DE2013/100172
(87) Internationale Veröffentlichungsnummer: WO 2013/170852

(56) Entgegenhaltungen:
- DE-A1- 19 718 535
- DE-A1-102007 052 866
- DE-A1-102008 022 968
- DE-U1- 9 210 457
- DE-U1-202004 006 726
- US-A- 5 347 894
- US-A1- 2005 075 650

## Beschreibung

Die Erfindung bezieht sich auf eine Kopfhalterung zur Fixierung des Kopfes von Patienten zur Erstellung von NMR-Aufnahmen so wie solchen, die auf der Applikation elektromagnetische Strahlen (Röntgen-, Gamma-) beruhen und/oder zur Durchführung chirurgischer Eingriffe mit einem C-Bogen entsprechend dem Oberbegriff des Anspruchs 1.

Die Erfindung bezieht sich auf eine Kopfhalterung, deren Zweck darin besteht, den Kopf eines Patienten bei der Erstellung von Aufnahmen nach der NMR- und/oder Röntgentechnik oder aber bei der Durchführung chirurgischer, insbesondere neuro-chirurgischer Eingriffe zu fixieren. Hierbei gilt insbesondere sicherzustellen, dass die nach der Fixierung des Kopfes regelmäßige Veränderung der Ausrichtung der Patientenliege im Wege der Verschwenkung zu Verschiebungen des Patienten unter dem Einfluss der Schwerkraft führt und dennoch die Fixierung des Kopfes beibehalten werden muss. Darüber hinaus ist zu gewährleisten, dass die Fixierung des Kopfes mit einem definierten Anpressdruck erfolgen kann. Die zu vorbekannten Zwecken einsetzbaren Kopfhalterungen bestehen deshalb in ihrem grundsätzlichen Aufbau aus einem C-Bogen, an dessen Enden jeweils wenigstens ein Dorn in der Weise angebracht wird, dass die beiden Dorne in diametraler Ausrichtung angeordnet sind, wobei einer der Dorne zur Fixierung des Kopfes im Wege der Anpassung an unterschiedliche anatomische Abmessungen axial bewegbar ist. Zur Erzeugung eines definierten Anpressungsdruckes wird wenigstens einer der Dorne an einem axial beweglichen Bolzen geführt, der seinerseits über eine Federbeaufschlagung eine axiale Beaufschlagung erfährt, wobei Anzeigen zur Wiedergabe des augenblicklichen Anpressdruckes vorhanden sind. Hierdurch wird eine Überprüfung und definierte Einstellung des Anpressdruckes möglich. Bei den Lösungen gattungsgemäßer Art erfolgt dies häufig dadurch, dass an den Bolzen in axialer Richtung nach außen zu weisend ein Stift befestigt ist, welcher den Gegenhalter der Feder bis nach außen zu durchgreift, wobei letztere über eine hohlzylindrische Schraubgewindehülse in ihrer relativ zur Achse des Bolzens definierten Lage verstellbar ist, sodass sich auf diese Weise der Anpressdruck der Feder verändert. Bei einer hohen Kompression der Feder wird der Abstand zwischen Bolzen und Gegenhalter kleiner, mit der Folge, dass der Stift um eine größere Strecke nach außen übersteht. Im umgekehrten Fall wird der Stift nur um eine dementsprechend kürzere Strecke überstehen. Somit ist die überstehende Länge des Stiftes ein unmittelbares Maß für den Anpressdruck des Bolzen und damit des Dornes am Kopf des Patienten. Zur Fixierung wird durch Drehen der Hülse der Anpressdruck so lange verändert, bis der definierte Wert angenommen ist. Eine entsprechende Offenbarung ist der DE 20 2004 006726 U1 und der DE 197 18 535 A1 entnehmbar, wo keine Fixiermittel zur temporären Festlegung des Bolzens vorzusehen sind, die eine Lösung der Spannvorrichtung als auch die Kraftanzeige von der Kopfhalterung erlauben.

Das dem Anmeldungsgegenstand am nächsten kommende Dokument US 2005/0075650 A21 offenbart bereits eine Kopfhalterung zur Fixierung des Kopfes von Patienten, bestehend aus einem C-Bogen, an dessen gegenüberliegendem Ende wenigstens ein Dorn befestigt ist und der Dorn an einem axial beweglichen Bolzen befestigt ist. Durch axiale Beaufschlagung mit einer Spannvorrichtung erfolgt unter Kontrolle einer Kraftanzeige das Festlegen des Bolzens am Kopf des Patienten. Auch ist hieraus bekannt, unter Beibehaltung der Fixierung des Bolzens, die Spannvorrichtung und die Kraftanzeige zu lösen.

Die Vorrichtungen gattungsgemäßer Art sind in mehrerer Hinsicht mit Nachteilen behaftet. Aufgrund der Notwendigkeit der relativen Bewegung einzelner mechanischer Teile gegeneinander, zu denen die axiale Verschieblichkeit des Bolzens, die Schraubverbindungen zur Einstellung des Federdruckes und die Relativbewegung der Hülse mit dem darin befindlichen Gegenhalter gehören, besteht die Notwendigkeit eines auch bei hoher Fertigungsgenauigkeit vorhandenen Spieles zwischen den relativ zu bewegenden Teilen. Dies führt dazu, dass bei einer Veränderung der auf den Dorn einwirkenden Kräfte bei Verstellung des Tisches Verschiebungen des Patienten auch nach erfolgter Fixierung unvermeidlich sind. Eine Verstärkung dieser Einflüsse erfolgt dadurch, dass die Druckanzeige aufgrund der Notwendigkeit eines Spieles für die Bewegung des Stiftes, wie auch der Feder, zusätzlich zu Ungenauigkeiten führen wird.
Weiterhin ist als Nachteil zu bezeichnen, dass die Außenabmessungen dieser auch während des chirurgischen Eingriffes vorhandenen Apparaturen, einen hohen Eigenplatzbedarf beanspruchen, der in nachteiliger Weise die Zugänglichkeit für den Operateur erschwert. Eine der Ursachen der großbauenden Anordnung ist die Notwendigkeit eines entsprechenden Federweges, der erforderlich ist, um den Dorn in die erforderlichen Positionen, also vom unbelasteten in den belasteten Zustand, überführen zu können.

Die der Erfindung zugrunde liegende Aufgabe besteht darin, derartige Kopfhalterungen dahingehend weiter zu entwickeln, dass sie die Fixierung des Kopfes auch bei Verschiebungen des Patienten unter dem Einfluss der Schwerkraft beibehalten..

Erfindungsgemäß wird diese Aufgabe durch die im Anspruch 1 angegeben Merkmale gelöst.

Diese Lösung wird im Kern durch das Vorhandensein zweier Merkmale bewirkt. Zum einen sind Fixiermittel vorgesehen, deren konkrete mechanische Realisierung im Rahmen der Erfindung grundsätzlich freisteht, sofern deren Sinn und Zweck, nämlich die vorübergehende Möglichkeit der Fixierung des Bolzens umsetzbar ist. Eine weitere entscheidende Lehre der Erfindung sieht die Möglichkeit vor, die Spannvorrichtung und die Kraftanzeige von der Kopfhalterung selbst zu lösen, wobei selbstredend der Begriff "Kopfhalterung" in diesem Zusammenhang nicht mehr Spannvorrichtung und Kraftanzeige umfassen soll. Auch hier ist im allgemeinsten Fall des Erfindungsgedankens die konkrete Ausbildung der lösbaren Befestigung von der Spannvorrichtung und/oder Kraftanzeige beliebig.

Der Querschnitt des Bolzens ist nach Art eines Polygons geformt, um einerseits das Erfordernis der Ausbildung einer Kante zu erfüllen und zum anderen dem Spannstein eine ebene Anpressfläche zur Verfügung zu stellen. Ohne Beschränkung der Allgemeinheit bieten sich hierfür die Dreieck-, Fünfeck-, Siebeneckformen an. Oberstes Ziel der Realisierung des Fixiermittels ist die Maximierung der Fixierung des Bolzens am C-Bogen.

Der erfindungsgemäße Aufbau erlaubt folgende Vorgehensweise: Die ersten Schritte bestehen in an sich bekannter Weise darin, dass der Kopf des Patienten in der Kopfhalterung positioniert wird, anschließend mindestens ein Dorn mit der Spannvorrichtung auf den Kopf des Patienten zu und zwar soweit bewegt wird, bis der gewünschte Anpressdruck erreicht wird. Die Überwachung des Spannvorganges bis zum Maximaldruck erfolgt über die Kraftanzeige. Neben dieser aus dem Stande der Technik bekannten und deshalb nicht weiter erläuterungsbedürftigen Vorgehensweise schließen sich in entscheidender Weise die folgenden beiden Schritte an. Mit Erreichen der gewünschten Anpresskraft wird der Bolzen und folglich der darin befestigte Dorn durch die Aktivierung eines Fixiermittels festgelegt. Das Ergebnis ist, dass die Position des Bolzens während der chronologisch sich anschließenden Arbeitsschritte raumfest bleibt. Eingangs wurde erläutert, dass bei dem im allgemeinen notwendigen Verkippen die Patienten unter dem Einfluss der Schwerkraft eine Änderung des Anpressdruckes erfahren, was in nachteiliger Weise nicht nur zu einer Änderung des Anpressdruckes an sich sondern auch bei bestimmten Spannvorrichtungen, zu denen die weitverbreiteten und mit Hilfe von Federkraft erzeugten Anpressdrucke gehören, zu einer Änderung der Position des Bolzens bzw. Dorns führen. Gleichermaßen trägt hierzu das in der Vorrichtung vorhandene Spiel bei. Die Fixierung hat nun den entscheidenden Vorteil, das die räumliche Position des Bolzens während der anschließenden Arbeitsphase sichergestellt bleibt und die bisherigen, zu Ungenauigkeiten bei bildgebenden Verfahren und/oder Abweichungen in der Positionierung bei operativen Eingriffen Anlass gebenden Einflüsse mit Sicherheit ausgeschlossen sind. Durch die Fixierung des Bolzens endet die Notwendigkeit der Kraftbeaufschlagung durch die Spannvorrichtung, nachdem der gewünschte Anpressdruck erreicht wurde und die Kraftanzeige in ihrer Funktion nicht mehr benötigt wird. Diese technische Situation erlaubt es einen weiteren entscheidenden Schritt zu tun, nämlich die Spannvorrichtung und Kraftanzeige von der (verbleibenden) Kopfhalterung zu trennen.
Um beim nächsten Patienten die vorbeschriebenen Arbeitsschritte durchführen zu können, müssen Spannvorrichtung und Kraftanzeige wieder an der Kopfhalterung befestigt werden können, sodass deren Abnahme vorübergehend und damit lösbar sein muss. Anschließend werden die Aufnahmen gefertigt und/oder die chirurgischen Eingriffe vorgenommen.

Die durch Realisierung der erfindungsgemäßen Lehre erreichbaren Vorteile sind vielfältig:
Die durch Applikation des Fixiermittels erfolgende Festlegung des Bolzens führt zu dessen völliger Spielfreiheit, sodass bei der Verstellung des Tisches oder der Trepanation jegliche zu Ungenauigkeiten führende Relativverschiebungen ausgeschlossen sind. Das Lösen von Spannvorrichtungen und Kraftanzeige verkürzt den Abstand zwischen Operateur und Patient und erleichtert demzufolge das Handling während des chirurgischen Eingriffs. Bei der Durchführung von NMR-Aufnahmen ist darauf zu achten, dass Spannvorrichtung und Kraftanzeige nicht zu Artefakten führen und im Bild wiedergegeben werden. Die Verwendung kostenintensiver Materialien sind die zwingende Konsequenz, was im Stande der Technik zum Einsatz von Keramikmaterialien geführt hat. Die gleichen Anforderungen gelten dann, wenn Aufnahmetechniken unter Anwendung elektromagnetischer Strahlen angewandt werden. Auch hier sind entsprechende, Artefakte vermeidende Aufnahmen das Ziel. Aufgrund der Entfernung von Spannvorrichtung und Kraftanzeige vor der Aufnahme wird die Möglichkeit eröffnet gängige und damit preisgünstige Materialien einzusetzen, die im Hinblick auf ihren funktionellen Möglichkeiten den bisher verwendeten Materialien überlegen sein können. Qualitätsmängel kommen auch dadurch zu Stande, dass bei einer mechanischen Umsetzung der Kraftanzeige sich zusätzlich zu denen der Spannvorrichtungen die Ungenauigkeiten in der Anzeige der Kraft addieren.

Die Querschnittsfläche des Bolzens nach Art eines Polygons trägt in erheblichem Masse zu einer Reduzierung der Spielfreiheit in azimutaler Richtung und folglich auch zu einer Verringerung der Spielfreiheit insgesamt bei.

In einer bevorzugten Ausgestaltung der Spannvorrichtung, die sich aufgrund der Einfachheit ihres Aufbaues und durch zuverlässige Betriebssicherheit auszeichnet, erfolgt eine Krafteinwirkung auf den Bolzen durch eine an der patientenfernen Stirnseite axial angreifende Schraubenfeder, deren gegenüberliegendes Ende an einem in axialer Richtung verstellbaren Gegenhalter anliegt. Die Kompression der Feder wird durch Verschiebbarkeit des Gegenhalters relativ zu dem die Führung für den Bolzen bildenden Gehäuse erzeugt. Wird der Gegenhalter in Richtung auf den Patienten zu bewegt, erfolgt eine Kompression der Feder, die zu einer Kraftentwicklung führt, die auf das Ende des Bolzens einwirkt und durch Übermittlung desselben mit gleicher Kraft den Dorn gegen den Kopf des Patienten presst. Bei einer Änderung des Abstandes des Gegenhalters weg vom Patienten entspannt sich die Feder und führt demzufolge zu einer Reduzierung der Anpresskraft des Dorns. Die Verstellung des Gegenhalters erlaubt eine kontinuierliche Einstellung der Anpresskraft.

Für die bauliche Realisierung des verstellbaren Gegenhalters werden zwei Möglichkeiten vorgeschlagen:
Zum einen ist der zur Fixierung des Kopfes des Patienten benötigte Gegenhalter ein Schraubstift, welcher mit einem an der Innenfläche des Gehäuses befindlichen Schraubgewinde kämmt. Die Stirnfläche des Schraubstiftes bildet den Gegenhalter der Feder.

In einer Alternativen wird der Gegenhalter durch einen Hohlzylinder gebildet, dessen äußerer Stirnfläche der Bildung des Gegenhalters dient. Der Hohlzylinder kämmt mit einem Innen-/ Außengewinde des Gehäuses und bewirkt durch Drehen die axiale Verstellung des Gegenhalters und demzufolge die Beeinflussung der durch die Feder ausgeübten Kraft.

Eine weitere Möglichkeit besteht darin, dass der Hohlzylinder ein Außengewinde aufweist, welches die Befestigung im C-Bogen durchgreift und das Gewinde mit einer Schraubenmutter kämmt, die patientenseitig auf den Hohlzylinder aufgeschraubt ist und am C-Bogen flächig zur Anlage kommt. Das Betätigen der Mutter bewirkt ein axiales Verschieben des Hohlzylinders und folglich auch des darin befindlichen Gegenhalters. Aufgrund des herrschenden Anpresszuges ist die Mutter von der Patientenseite auf den Hohlzylinder aufzubringen.

Im vorbeschriebenen Fall der Herleitung der Spannkräfte aus einer komprimierten Feder empfiehlt die Erfindung als Kraftanzeige die Verwendung eines Stiftes, der mit seinem einen Ende direkt oder indirekt mit dem jeweiligen Ende des Bolzen zusammenarbeitet und über den Gegenhalter nach außen geführt ist, wobei die Ausrichtung des Stiftes in Bewegungsrichtung des Bolzens erfolgt. Bei einer Kompression der Feder ändert sich der Abstand zwischen Bolzen und Gegenhalter mit dem Ergebnis, dass der Stift umso mehr nach außen übersteht. Bei Nachlassen der Kompression erhöht sich der Abstand zwischen Bolzen und Gegenhalter mit dem Ergebnis, dass der Stift nur noch um eine kleinere Länge übersteht. Die Länge des nach außen überstehenden Endes des Stiftes gilt als Anzeige für die durch die Feder und folglich auch durch den Bolzen mit dem daran befindlichen Dorn auf den Patienten ausgeübte Kraft.

Die konkrete Ausgestaltung des Fixiermittels der Erfindung ist im Rahmen der Erfindung weitgehend beliebig, sofern es den Zweck der temporären Festlegung des Bolzens erfüllt. Der Bolzen muss einerseits während der Phase der Einspannung des Kopfes in axialer Richtung verschiebbar sein, jedoch später, wenn die gewünschte Anpresskraft erreicht wurde als auch in der sich daran anschließenden Arbeitsphase (Erstellen der Aufnahme und/oder Durchführen des chirurgischen Angriffes) festlegbar sein.
Eine bevorzugte Realisierung des Fixiermittels kann durch einen Spannstein erfolgen, welcher im wesentlichen in radialer Richtung auf den Bolzen zu bewegt wird und hierbei eine Anpressung bewirkt, die aufgrund der Führung des Bolzens ein temporäres Verklemmen und damit Festlegen des Bolzens zum Ergebnis hat. Auf welche Art und Weise die Bewegung des Spannsteins erfolgt, ist im Rahmen der Erfindung letztlich beliebig. Entscheidend ist die Erzeugung einer Klemmung, die zur Spielfreiheit führt.

In einer vorteilhaften Weitergestaltung ist der Querschnitt des Bolzens auf der dem Spannstein gegenüberliegenden Seite als Kante geformt und arbeitet mit einer komplementär dazu gestalteten Führung zusammen. Mit Betätigen des Spannsteines wird die Kante in die komplementär geformte Nut in der Führung bis zur flächigen Anlage eingepresst und dadurch ein Festlegen des Bolzens sowohl bei azimutaler als auch bei axialer Bewegung des Bolzens bewirkt. Durch Lösen der Verbindung durch eine radiale Bewegung des Spannsteines weg vom Bolzen wird dieser freigegeben und kann erneut axial frei verschoben werden.

Als zusätzliche Maßnahme der Fixierung können auf dem Spannstein Schraubennuten und demgegenüber auf dem Bolzen komplementär hierzu geformte Gewinde eingebracht sein, die derart dimensioniert sind, dass sie gegenseitig in Eingriff gebracht werden können. Durch diese Maßnahme kann eine weitere Fixierung des Bolzens in Abhängigkeit von der Orientierung der Nuten sowohl bei axialen und/oder azimutalen Bewegungen des Bolzens unterbunden werden. Bei einem ohne Steigung umlaufenden Gewinde, bei denen die Nuten also nur in azimutaler Richtung orientiert sind, wird die axiale Bewegung des Bolzens unterbunden. Bedingt durch die Geometrie der Verzahnung kann das Zusammenwirken von Spannstein und Bolzen nur in diskreten Schritten vorgenommen werden. Bei hinreichender Feinheit der Verzahnung, d. h. bei entsprechend schmalen Zähnen wird dieser Nachteil akzeptabel.

Die Anzahl der die Berührungspunkte am Kopf des Patienten definierende Dorne ist im Rahmen der Erfindung grundsätzlich beliebig. Erforderlich sind wenigstens zwei, im wesentlichen diametral einan der gegenüberliegende Dorne zur Fixierung des Kopfes. Dabei wird in der Regel der eine Dorn starr, also raumfest, der gegenüberliegende Dorn jedoch mit der Spannvorrichtung zur Erzeugung der definierten Anpresskraft versehen sein. Eine Federbeaufschlagung beider Dorne gibt wenig Sinn, da in der Phase der Fixierung und Erzeugung des definierten Anpressdruckes die Erhöhung der Kraft auf einer Seite zwangsläufig ein Nachgeben der federbelasteten Spannvorrichtung auf der anderen Seite zur Folge hat, was eine Ungenauigkeiten in der Positionierung verursachende Verschiebung der Position des Kopfes zum unerwünschten Ergebnis hätte.

Ausdrücklich ist es möglich, dass der einzelne Dorn durch eine als Wippe ausgebildete mit mehreren Dornen versehene Baueinheit ersetzt wird. Die Wirkungslinie der Wippe wird beschrieben durch die Mittelsenkrechte zwischen den Dornen. Der Begriff "Wippe" beinhaltet die Möglichkeit einer um kleine Winkel verschwenkbare Anbringung.

Zusätzlich zu den beiden im wesentlichen diametral einander zugeordneten Dornen sind weitere, im wesentlichen hiervon abweichend ausgerichtete Dornen zur zusätzlichen Fixierung des Kopfes denkbar.

Im Hinblick auf die Materialien schließlich wird vorgeschlagen, dass der Bolzen so wie dessen Führung aus transluzenten Materialien bestehen, die sich bei NMR-Anwendung und/oder für die Abbildung mit Hilfe elektromagnetischer Wellen (z. B. Röntgen-Gamma) eignen. Entscheidend ist, dass die Spannvorrichtung und die Kraftanzeige in der Wahl des Materials beliebig bleiben. Die Entstehung von Artefakten sowie Ungenauigkeiten in der Positionierung werden dann zuverlässig eliminiert.

Im Hinblick auf die Handhabung schließlich erweist sich als Vorteil, wenn die Spannvorrichtung als auch die Kraftanzeige in einem gemeinsamen Gehäuse untergebracht sind, weil dann mit dem minimalen Aufwand des Lösens der Befestigung des Gehäuses von der übrigen Vorrichtung bereits deren vollständige Entfernung vorgenommen werden kann.

Weitere Einzelheiten, Merkmale und Vorteile der Erfindung lassen sich dem nachfolgenden Beschreibungsteil entnehmen, indem anhand der Zeichnung ein Ausführungsbeispiel der Erfindung näher erläutert ist. Es zeigen:
- Figur 1:: eine Draufsicht in Richtung der Körperachse eines Patienten
- Figur 2:: einen axialen Längsschnitt durch eine erfindungsgemäße Vorrichtung.

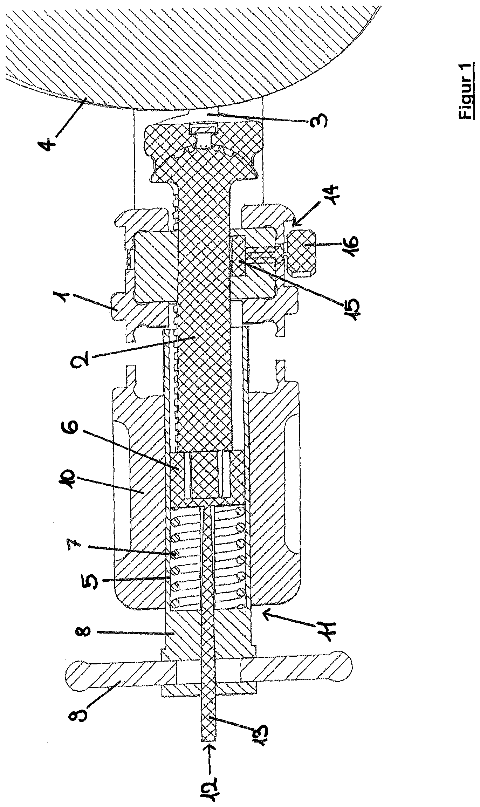

In Figur 1 ist eine schematisch gehaltene Darstellung einer erfindungsgemäßen Kopfhalterung bei eingespanntem Kopf des Patienten in einer sich durch Draufsicht in Richtung der Körperlängsachse des Patienten sich ergebenden Seitenansicht. Der grundsätzliche Aufbau wird in an sich bekannter Weise durch den C-Bogen 1 gebildet. Hierin ist im wesentlichen mittig der Kopf 4 eines Patienten festgelegt und der C-Bogen 1 liegt in einer axialen Schnittebene im Hinblick auf die Längsachse des Körpers bzw. Kopfes des Patienten. Im linken Endbereich des C-Bogens1 ist die erfindungsgemäße Festlegung des Kopfes wiedergegeben, die beginnend vom Kopf 4 des Patienten aus zwei am Kopf 4 mit einer definierten Kraft und symmetrisch zu einer Mittelachse angeordneten Dornen 3 besteht. Die definierte Anpresskraft wird dadurch erzeugt, dass die Dornen 3 an einem axial beweglichen Bolzen 2 befestigt sind, der in der gezeigten Draufsicht nur mit seinem äußeren Ende zu erkennen ist, da die Restlänge durch weitere Bauteile im wesentlichen durch das Gehäuse 10 abgedeckt ist. Die Festlegung des axial verschiebbaren Bolzens 2 erfolgt über eine im Endbereich des C-Bogens 1 senkrecht zum Bolzen 2 verlaufenden Schraube 16. Wird die Schraube 16 eingedreht, wird der Bolzen 2 festgelegt und es entsteht Spielfreiheit. Das sich anschließende, im wesentlichen hohlzylinderartig geformte Gehäuse 10 ist mit einem segmentartig verlaufenden Schlitz versehen, in welchem die Schraube 16 verläuft, wobei der Schlitz die Möglichkeit eröffnet, dass das Gehäuse 10 in radialer Richtung von der Vorrichtung abgezogen werden kann.

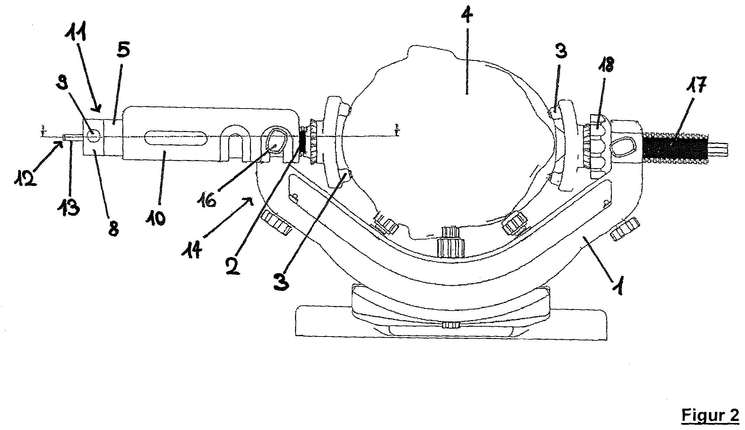

An der dem Bolzen 2 gegenüberliegenden Ende des Gehäuses 10 erkennt man einen Gegenhalter 8, der nach Art eines stirnseitig verschlossenen Hohlzylinders 5 geformt ist und in dessen Inneren die aufgrund der Seitenansicht nicht wiedergegebene Schraubenfeder 7 angeordnet ist. Am äußeren Ende des Zylinders bzw. Gegenhalters 8 ist ein senkrecht zur Zeichenebene verlaufender Betätigungsgriff 9 wiedergegeben, mit dessen Hilfe eine Schraubbewegung des Gegenhalters 8 bzw. des diesen bildenden Hohlzylinders 5 relativ zum C-Bogen 1 bzw. Bolzen 2 möglich ist. Nach außen zu steht ein Stift 13 in axialer Richtung über, der an seiner Oberfläche mit einer Kraftanzeige 12 versehen ist. Die axiale Verschieblichkeit des Stiftes 13 dient als Kraftanzeige 12 des vermittels des Bolzens 2 und der Dorne 3 auf den Kopf 4 ausgeübten Kraft. Das Zusammenwirken der einzelnen Elemente ist aus der nachfolgend in Figur 2 wiedergegeben Darstellung deutlicher zu erkennen. Mithilfe des Gehäuses 10 wird es möglich Spannvorrichtung 11 und Kraftanzeige 12 bei festgelegten Bolzen 2 zu entfernen, wobei die Schraube 16 bzw. die Fixiermittel 14 und ebenso der festgelegte Bolzen 2 fest mit dem C-Bogen 1 verbunden bleiben, mit dem Ergebnis, dass der Anpressdruck trotz der Beseitigung der Spannvorrichtung 11 auf dem Kopf 4 des Patienten erhalten bleibt.

Der soeben beschriebenen, über einen axial verschiebbaren Bolzen 2 auf der linken Seite einen definierten Anpressdruck erzeugenden Teil der Fixierung gegenüber liegt eine konventionelle, eine Abstützung bildende weitere Fixierung, die ausgehend vom Kopf 4 aus den Dornen 3 besteht, die an einem ebenfalls axial bewegbaren Gegenbolzen 17 befestigt sind, wobei dieser in axialer Richtung relativ zum C-Bogen 1 verschieblich ist. Die axiale Fixierung erfolgt hier über eine Überwurfmutter 18, die mit den Gewinde des Gegenbolzens 17 kämmt und von der Patientenseite her am C-Bogen 1 festliegt. Im Ergebnis erhält man eine starre Fixierung der rechtsseitigen Dorne 3. Eine Veränderung der Anpresskraft ist durch Betätigen der Gegenmutter 18 grundsätzlich möglich, jedoch lässt sich keine definierte Spannkraft einstellen. Aufgabe der Überwurfmutter 18 ist eine Anpassung an die konkreten anatomischen Gegebenheiten des zu untersuchenden Patienten.

Die Funktion der in Figur 1 beschriebenen Vorrichtung lässt sich wesentlich deutlicher anhand der Darstellung der Figur 2 erkennen. Die nur als Teilausschnitt wiedergegebene Vorrichtung ist in der Phase der Anlage am Kopf des Patienten gezeigt. Entsprechend den Regeln des technischen Zeichnens sind die Schnittflächen in Schraffur wiedergegeben. Der etwa halbkreisförmig verlaufende C-Bogen 1 ist senkrecht zur Zeichenebene orientiert und umgreift den Kopf 4 des Patienten. Im Endbereich ist axial verschieblich der in der Zeichenebene verlaufende Bolzen 2 gezeigt, der an seinem äußeren Ende mit einem Dorn 3 versehen ist, der seinerseits am Kopf 4 des Patienten anliegt und somit den eingespannten Zustand anzeigt.

Das dem Patienten gegenüberliegende Ende des Bolzens 2 greift in einen in einem Hohlzylinder 5 verschiebbar gelagerten Kolben 6 ein. An der dem Bolzen 2 gegenüberliegenden Stirnflächen presst eine Schraubenfeder 7 den Kolben 6 gegen die Stirnfläche des Bolzens 2. Damit wirkt der Kolben 6 als Kraftvermittler zwischen Schraubenfeder 7 und Bolzen 2, wobei der jeweilige Kontakt durch loses Aufliegen hergestellt ist.

Der Hohlzylinder 5 ist an seinem dem Bolzen 2 gegenüberliegenden Ende stirnseitig verschlossen und bildet auf diese Weise den Gegenhalter 8 für die Schraubenfeder 7. Wie über den Betätigungsgriff 9 angezeigt, ist der Hohlzylinder 5 über ein Gewinde innerhalb des Gehäuses 10 geführt, sodass bei einem Eindrehen des Hohlzylinders die Schraubenfeder 7 eine Komprimierung erfährt und folglich den Kolben 6 mit verstärkter Kraft gegen den Bolzen 2 drückt, der wiederum seinerseits den Dorn 3 an den Kopf 4 des Patienten anpresst. Bei einem Drehen des Betätigungsgriffes 9 in die Gegenrichtung führt dies zu einer Entlastung der Schraubenfeder 7 mit der Folge, dass nach den vorbeschriebenen Wirkmechanismen die Anpresskraft des Dornes 3 reduziert wird. Die Anordnung bestehend aus Bolzen 2 mit daran befestigtem Dorn 3, Kolben 6, Schraubenfeder 7, sowie den gleichzeitig den Gegenhalter 8 bildeten Hohlzylinder 5 beschreiben in ihrer Gesamtheit die Spannvorrichtung 11.

Zur Ausübung und zur Kontrolle einer definierten Anpresskraft des Dornes 3 am Kopf 4 des Patienten ist eine Kraftanzeige 12 vorgesehen, die auf mechanische Art und Weise erfolgt und durch einen Stift 13 als Anzeige gebildet wird, der koaxial und in Verlängerung zum Bolzen 2 verläuft, am Kolben 6 befestigt ist, die Schraubenfeder 7 durchgreift und über eine geeignete, der Führung dienende Öffnung nach außen zu übersteht. Die Länge des sichtbaren Stiftes 13 kann als Anzeige der Position des Kolbens 6 unmittelbar durch die Schraubenfeder 7 ausgeübte und durch Vermittlung des Kolbens 2 auf den Dorn 3 ausgeübte Kraftanzeige beschreiben. Entsprechende Indexmarkierungen erleichtern die zahlenmäßige Zuordnung. Die gewünschte Krafteinstellung lässt sich auch in reproduzierbarer Weise wiederholen.

Mit Erreichen des gewünschten Anpressdruckes und damit der Endposition des Dornes 3 ist als ein wesentlicher Gedanke der Erfindung ein Fixiermittel 14 vorgesehen, welches in gezeigten Ausführungsbeispiel aus einem Spannstein 15 besteht, der über eine von außen betätigbare Schraube 16 in radialer Richtung zum Bolzen 2 hin bewegt wird und sich hiergegen presst. Der Spannstein 15 hat dabei mit einer solchen Kraft gegen den Bolzen 2 in Anlage zu kommen, dass der Bolzen 2 unter dem Einfluss der auf ihn einwirkende Kräfte raumfest in C-Bogen 1 festgelegt bleibt. Eine Verstärkung der Fixierung bewirken Verzahnungen, die einerseits am Bolzen 2 und andererseits an der zur Anlage kommenden Fläche des Spannsteins 15 angeformt sind.

Gemäß einem Kerngedanken vorliegender Erfindung lässt sich die Spannvorrichtung 11 mit der Kraftanzeige 12, die gemeinsam in einen Gehäuse 10 getragen werden, nach Lösen entsprechender, hier nicht gezeigter Verbindungen von dem C-Bogen 1 und dem darin festgelegten Bolzen 2 problemlos abziehen. Der in der Zeichnung wiedergegebene Abstand zwischen C-Bogen1 und Gehäuse 10 verdeutlicht das Vorhandensein zweier selbstständiger Bauteile, wobei ausdrücklich zu ergänzen ist, dass der Bolzen 2 lediglich in loser Anlage zum Kolben 6 steht.
Nach der Abnahme von Spannvorrichtung 11 und Kraftanzeige 12 lassen sich die erforderlichen Arbeitsmaßnahmen in Form der Erstellung von Aufnahmen und/oder der Durchführung chirurgischer Eingriffe vornehmen.

Im Ergebnis erhält man eine spielfreie und kleinbauende Anordnung, die gegenüber dem Stande der Technik erhebliche Vorteile bietet.

### Bezugszeichenliste

- 1: C-Bogen
- 2: Bolzen
- 3: Dorn
- 4: Kopf
- 5: Hohlzylinder
- 6: Kolben
- 7: Schraubenfeder
- 8: Gegenhalter
- 9: Betätigungsgriff
- 10: Gehäuse
- 11: Spannvorrichtung
- 12: Kraftanzeige
- 13: Stift
- 14: Fixiermittel
- 15: Spannstein
- 16: Schraube
- 17: Gegenbolzen
- 18: Überwurfmutter

## Patentansprüche

1. Kopfhalterung zur Fixierung des Kopfes von Patienten zur Erstellung von NMR-Aufnahmen so wie solchen, die auf der Applikation elektromagnetische Strahlen (Röntgen-, Gamma-) beruhen und/oder zur Durchführung chirurgischer Eingriffe
- mit einem C-Bogen (1), an dessen Endbereich jeweils wenigstens ein Dorn (3) befestigt ist, die einander im wesentlichen diametral zugeordnet sind, wobei wenigstens ein Dorn (3) koaxial an einem axial beweglichen Bolzen (2) befestigt ist, einer Spannvorrichtung (11), durch die die Bolzen (2) axial beaufschlagbar ist sowie eine der Spannvorrichtung zugeordnete Kraftanzeige (12), und Fixiermittel (14) vorhanden sind, die der temporären Festlegung des Bolzens (2) dienen und dass bei aktiviertem Fixiermittel die Befestigung von Spannvorrichtung (11) und Kraftanzeige (12) an der Kopfhalterung lösbar ist **dadurch gekennzeichnet, dass** das Fixiermittel (14) ein in radialer Richtung zum Bolzen (2) bewegbarer und zur Anlage am Bolzen (2) kommender Spannstein (15) zur Herstellung einer Klemmwirkung ist und der Querschnitt des Bolzens (2) im Bereich des Spannsteins (15) nach Art eines Polygons gewählt ist.

2. Kopfhalterung nach Anspruch 1, **dadurch gekennzeichnet, dass** auf den Bolzen (2) eine sich über einen Gegenhalter (8) abstützende Schraubenfeder (7) einwirkt und der Gegenhalter (8) axial verstellbar ist.

3. Kopfhalter nach Anspruch 2, **dadurch gekennzeichnet, dass** der Gegenhalter (8) die Stirnfläche eines Schraubstiftes ist, der mit der Innenfläche des Gehäuses (10) kämmt.

4. Kopfhalter nach Anspruch 2, **dadurch gekennzeichnet, dass** der Gegenhalter (8) die äußere Stirnfläche eines Hohlzylinders ist, welcher relativ zum Gehäuse (10) verstellbar ist.

5. Kopfhalter nach Anspruch 4, **dadurch gekennzeichnet, dass** der mit seiner Stirnfläche dem Gegenhalter (8) bildende Hohlzylinder (5) ein Außengewinde aufweist, welches die Befestigung im C-Bogen1 durchgreift und auf das Gewinde eine Schraubenmutter patientenseitig aufgeschraubt ist, die am C-Bogen1 flächig anliegt.

6. Kopfhalter nach einem der Ansprüche 1 bis 5, **dadurch gekennzeichnet, dass** ein Stift (13) direkt oder indirekt mit dem Bolzen (2) in Verbindung steht und über den Gegenhalter (8) nach außen zu in Bewegungsrichtung des Bolzens (2) geführt ist.

7. Kopfhalter nach einem der vorgehenden Ansprüche, **dadurch gekennzeichnet, dass** der Querschnitt des Bolzens (2) auf der dem Fixiermittel (14) gegenüberliegenden Seite als in axialer Richtung verlaufende Kante geformt ist und mit der hierzu komplementär gestalteten Führung des Gehäuses (10) zusammenarbeitet.

8. Kopfhalter nach Anspruch 7, **dadurch gekennzeichnet, dass** auf der Kontaktfläche des Spannsteins (15) und/oder der des Bolzens (2) oberflächig Nuten angebracht sind.

9. Kopfhalter nach Anspruch 8, **dadurch gekennzeichnet, dass** die Orientierung der Nuten senkrecht zur Bewegungsrichtung des Bolzens (2) gerichtet sind.

10. Kopfhalter nach einem der vorhergehenden Ansprüche, **dadurch gekennzeichnet, dass** ausschließlich den Dornen (3) einer Seite eine Spannvorrichtung (11) zugeordnet ist.

11. Kopfhalter nach einem der vorhergehenden Ansprüche, **dadurch gekennzeichnet, dass** ein Dorn (3) durch eine mit mehreren Dornen (3) bestückte Wippen ersetzt ist.

12. Kopfhalter nach einem der vorhergehenden Ansprüche, **dadurch gekennzeichnet, dass** zusätzlich zu den diamentral zueinanderliegenden Dornen (3) weitere Dorne angebracht sind.

13. Kopfhalter nach einem der vorhergehenden Ansprüche, **dadurch gekennzeichnet, dass** der Bolzen (2) unter dessen Führung im C-Bogen (1) aus transluzentem Material bestehen.

14. Kopfhalter nach einem der vorhergehenden Ansprüche, **dadurch gekennzeichnet, dass** Spannvorrichtung und Kraftanzeige in einem gemeinsamen Gehäuse (10) untergebracht sind.

## Claims

1. Head holder for fixation of the head of patients for producing NMR images such as those which are based on the application of electromagnetic beams (X-rays, gamma rays) and/or for carrying out surgical procedures, having a C-bend (1), at each end region of which at least one mandrel (3) is fastened, these being substantially diametrically assigned with respect to each other, wherein at least one mandrel (3) is coaxially fastened to an axially moveable bolt (2) which can be acted upon in the axial direction via a clamping device (11), as well as a force indicator (12) assigned to the clamping device, and fixation means (14) are provided which serve to temporarily arrest the bolt (2) and that the fastening of the clamping device (11) and force indicator (12) to the head holder is detachable while the fixation is retained, **characterised in that** the fixation means (14) is a clamping block (15), which is movable in the radial direction towards the bolt (2) and comes into contact against the bolt (2), in order to produce a clamping effect and the cross-section of the bolt (2) is chosen in a polygonal form.

2. Head holder according to claim 1, **characterised in that** a helical spring (7), which is supported via a counter-holder (8), acts on the bolt (2) and the counter-holder (8) is axially adjustable.

3. Head holder according to claim 2, **characterised in that** the counter-holder (8) is the end face of a screw pin, which meshes with the inner surface of the housing (10).

4. Head holder according to claim 2, **characterised in that** the counter-holder (8) is the outer end face of a hollow cylinder, which is adjustable relative to the housing (10).

5. Head holder according to claim 4, **characterised in that** the hollow cylinder (5), which, with its end face, forms the counter-holder (8), has an external thread, which passes through the fastening in the C-bend and onto the thread, there is screwed, at the patient side, a nut, which lies in areal contact with the C-bend 1.

6. Head holder according to one of claims 1 to 5, **characterised in that** a pin (13) is in direct or indirect connection with the bolt (2) and, via the counter-holder (8), is guided outwardly in the direction of movement of the bolt (2).

7. Head holder according to one of the preceding claims, **characterised in that** the cross-section of the bolt (2), on the side opposite the fixation means (14), is formed as an edge running in the axial direction and cooperates with the guide, which is shaped such that it is complementary thereto, of the housing (10).

8. Head holder according to claim 7, **characterised in that** grooves are applied superficially on the contact surface of the clamping block (15) and/or on the contact surface of the bolt (2).

9. Head holder according to claim 8, **characterised in that** the orientation of the grooves is perpendicular to the direction of movement of the bolt (2).

10. Head holder according to one of the preceding claims, **characterised in that** a clamping device (11) is only assigned to the mandrels (3) of one side.

11. Head holder according to one of the preceding claims, **characterised in that** a mandrel (3) is replaced by a rocker equipped with a plurality of mandrels (3).

12. Head holder according to one of the preceding claims, **characterised in that** in addition to the mandrels (3) that lie diametrically with respect to one another, further mandrels are attached..

13. Head holder according to one of the preceding claims, **characterised in that** the bolt (2), as well as the guide in the C-bend (1), consist of translucent material.

14. Head holder according to one of the preceding claims, **characterised in that** the clamping device and force indicator are accommodated in a common housing (10).

## Revendications

1. Fixation de la tête destinée à maintenir la tête de patients de façon à réaliser des prises de résonance magnétique nucléaire (RMN) telles que celles qui reposent sur l'application de rayons électromagnétiques (rayons X, gamma) et/ou destinée à réaliser des interventions chirurgicales
- avec un coude en C (1), à l'extrémité duquel est fixé à chaque fois au moins un tenon (3), qui sont pour l'essentiel disposés diamétralement l'un par rapport à l'autre, sachant qu'au moins un tenon (3) est fixé de façon coaxiale sur un boulon (2) mobile dans le sens axial, un dispositif de serrage (11) par l'intermédiaire duquel le boulon (2) peut être alimenté dans le sens axial, ainsi qu'un affichage de force (12) attribué au dispositif de serrage et un moyen de fixation (14) étant présents, lesquels servent à fixer temporairement le boulon (2) et lorsque le moyen de fixation est activé, la fixation du dispositif de serrage (11) et de l'affichage de force (12) sur la fixation de la tête peut être détachée, **caractérisée par le fait que** le moyen de fixation (14) est un bloc de serrage (15) pouvant être déplacé dans le sens radial par rapport au boulon (2) et venant vers l'installation sur le boulon (2) afin de créer un effet de serrage, la section du boulon (2) dans la zone du bloc de serrage (15) étant choisie d'après un type de polygone.

2. Fixation de la tête selon la revendication 1, **caractérisée par le fait que** sur le boulon (2) agit un ressort hélicoïdal (7) s'appuyant sur un contre-appui (8), et que ce contre-appui (8) peut être réglé dans le sens axial.

3. Fixateur de tête selon la revendication 2, **caractérisé par le fait que** le contre-appui (8) est la surface frontale d'une tige à visser, qui se peigne avec la surface intérieure du boîtier (10).

4. Fixateur de tête selon la revendication 2, **caractérisé par le fait que** le contre-appui (8) est la surface frontale extérieure d'un cylindre creux, qui peut être déplacée par rapport au boîtier (10).

5. Fixateur de tête selon la revendication 4, **caractérisé par le fait que** le cylindre creux (5) formant le contre-appui (8) avec sa surface frontale présente un filetage extérieur, saisit la fixation dans le coude en C (1) et qu'un écrou à visser est vissé sur le filetage du côté du patient qui est posé en surface sur le coude en C (1).

6. Fixateur de tête selon une des revendications 1 à 5, **caractérisé par le fait qu'**une tige (13) est directement ou indirectement en liaison avec le boulon (2) et est guidée via le contre-appui (8) vers l'extérieur dans le sens du déplacement du boulon (2).

7. Fixateur de tête selon une des revendications précédentes, **caractérisé par le fait que** la section du boulon (2) prend, sur le côté opposé au moyen de fixation (14), la forme d'un bord courant dans le sens axial et fonctionne avec le guidage du boîtier (10) qui lui est de forme complémentaire.

8. Fixateur de tête selon la revendication 7, **caractérisé par le fait que** des rainures sont disposées superficiellement sur la surface de contact du bloc de serrage (15) et/ou celle du boulon (2).

9. Fixateur de tête selon la revendication 8, **caractérisé par le fait que** l'orientation des rainures est dirigée verticalement par rapport au sens du déplacement du boulon (2).

10. Fixateur de tête selon une des revendications précédentes, **caractérisé par le fait qu'**un dispositif de serrage (11) est exclusivement attribué aux tenons (3) d'un côté.

11. Fixateur de tête selon une des revendications précédentes, **caractérisé par le fait qu'**un tenon (3) est remplacé par une bascule munie de plusieurs tenons (3).

12. Fixateur de tête selon une des revendications précédentes, **caractérisé par le fait que** d'autres tenons sont posés en sus des tenons (3) placés diamétralement les uns par rapport aux autres.

13. Fixateur de tête selon une des revendications précédentes, **caractérisé par le fait que** le boulon (2) consiste en un matériau translucide en dessous de son guidage dans le coude en C (1).

14. Fixateur de tête selon une des revendications précédentes, **caractérisé par le fait que** le dispositif de serrage et l'affichage de force sont logés dans un boîtier (10) commun.
